# EUROPEAN PATENT APPLICATION

(11) **EP 4 613 228 A1**
(43) Date of publication of application: **10.09.2025**
(21) Application number: 25157360.6
(22) Date of filing: 12.02.2025
(51) Int. Cl.: A61B 34/30, A61B 34/37, A61B 34/00

(54) **ROBOTIC SURGICAL SYSTEM, CONTROL METHOD FOR ROBOTIC SURGICAL SYSTEM, PROGRAM, AND STORAGE MEDIUM**

(30) Priority: 01.03.2024 JP 2024030883
(71) Applicant: KAWASAKI JUKOGYO KABUSHIKI KAISHA, Kobe-shi, Hyogo 650-8670 (JP)
(72) Inventor: KODAMA, Kazuki, Kobe-shi, Hyogo, 650-8670 (JP); YAMAMOTO, Daisuke, Kobe-shi, Hyogo, 650-8670 (JP)
(74) Representative: Hoffmann Eitle

(57) **Abstract**

A robotic surgical system (500) includes an operation apparatus (200) including an operation unit (110) to receive an operation for a surgical instrument (1), the operation unit having a coordinate system (C1) rotated by a predetermined angle (θ) with respect to a coordinate system (C13) of a surgical apparatus (100), and a receiver (130) to receive a change in the predetermined angle by an operator.

## Description

### BACKGROUND OF THE INVENTION

### Field of the Invention

The present disclosure relates to a robotic surgical system, a control method for a robotic surgical system, a program, and a storage medium.

### Description of the Background Art

Conventionally, a robotic surgical system including a robot arm to which a surgical instrument is attached is known. U.S. Patent No. 6,424,885 discloses a robotic surgical system including a robot arm and a master control console to operate the robot arm. The master control console includes a master controller to be operated by the hand of an operator and a viewer into which the head of the operator is inserted. The angle of the viewer with respect to a horizontal plane is adjustable. In U.S. Patent No. 6,424,885, the coordinate system of the master controller is automatically adjusted according to the adjusted angle of the viewer. For example, the viewer is adjusted to an angle at which the operator looks down, and the coordinate system of the master controller is adjusted according to the angle of the viewer adjusted to the angle at which the operator looks down.

However, in U.S. Patent No. 6,424,885, the coordinate system of the master controller is automatically adjusted according to the angle of the viewer, and thus the operability of the master controller may conceivably be poor depending on the operator. Therefore, it is desired to improve the operability of the master controller.

### SUMMARY OF THE INVENTION

The present disclosure is intended to provide a robotic surgical system, a control method for a robotic surgical system, a program, and a storage medium that each enable improved operability of an operation unit.

A robotic surgical system according to a first aspect of the present disclosure includes a surgical apparatus including a robot arm to allow a surgical instrument to be attached thereto, an operation apparatus including an operation unit to receive an operation for the surgical instrument, the operation unit having a coordinate system rotated by a predetermined angle with respect to a coordinate system of the surgical apparatus, a controller configured or programmed to perform a control to move the surgical instrument by the robot arm based on the operation received by the operation unit, and a receiver to receive a change in the predetermined angle by an operator.

In the robotic surgical system according to the first aspect of the present disclosure, the receiver is configured to receive the change in the predetermined angle by the operator. Accordingly, the rotation angle of the coordinate system of the operation unit with respect to the coordinate system of the surgical apparatus can be changed, and thus the coordinate system of the operation unit can be adjusted such that the operator can easily operate the operation unit. Consequently, the operability of the operation unit can be improved.

A control method for a robotic surgical system according to a second aspect of the present disclosure includes receiving a change by an operator in a predetermined angle of a coordinate system of an operation unit rotated by the predetermined angle with respect to a coordinate system of a surgical apparatus, changing the predetermined angle of the coordinate system of the operation unit based on a received change in the predetermined angle, receiving an operation with the operation unit for a surgical instrument attached to a robot arm in a state in which the coordinate system of the operation unit has been changed, and moving the surgical instrument by the robot arm based on a received operation.

As described above, the control method for a robotic surgical system according to the second aspect of the present disclosure includes receiving the change in the predetermined angle by the operator, and changing the predetermined angle of the coordinate system of the operation unit based on the received change in the predetermined angle. Accordingly, the rotation angle of the coordinate system of the operation unit with respect to the coordinate system of the surgical apparatus can be changed, and thus the coordinate system of the operation unit can be adjusted such that the operator can easily operate the operation unit. Consequently, it is possible to provide the control method for a robotic surgical system that enables improved operability of the operation unit.

According to the robotic surgical system, the control method for a robotic surgical system, the program, and the storage medium according to the present disclosure, the operability of the operation unit can be improved.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

FIG. 1 is a diagram showing the configuration of a robotic surgical system according to an embodiment.
FIG. 2 is a diagram showing a display of a medical cart according to the embodiment.
FIG. 3 is a diagram showing the configuration of the medical cart according to the embodiment.
FIG. 4 is a diagram showing the configuration of a robot arm according to the embodiment.
FIG. 5 is a diagram showing a pair of forceps.
FIG. 6 is a perspective view showing the configuration of an arm operation unit according to the embodiment.
FIG. 7 is a diagram for illustrating translational movement of the robot arm.
FIG. 8 is a diagram for illustrating rotational movement of the robot arm.
FIG. 9 is a diagram showing an endoscope.
FIG. 10 is a diagram showing a pivot position setting instrument.
FIG. 11 is a diagram showing operation units according to the embodiment.
FIG. 12 is a diagram showing a right-handed wrist according to the embodiment.
FIG. 13 is a diagram showing a left-handed wrist according to the embodiment.
FIG. 14 is a perspective view showing foot pedals according to the embodiment.
FIG. 15 is a control block diagram of the robotic surgical system according to the embodiment.
FIG. 16 is a control block diagram of the robot arm according to the embodiment.
FIG. 17 is a control block diagram of a positioner and the medial cart according to the embodiment.
FIG. 18 is a control block diagram of the operation unit according to the embodiment.
FIG. 19 is a diagram for illustrating a method for calculating an axis value of each axis.
FIG. 20 is a diagram showing a base coordinate system and a tool coordinate system with respect to the endoscope.
FIG. 21 is a diagram showing an endoscope coordinate system.
FIG. 22 is a diagram showing a display and the endoscope coordinate system.
FIG. 23 is a diagram showing the coordinate system of the operation unit.
FIG. 24 is a diagram showing the coordinate system of the operation unit and the endoscope coordinate system.
FIG. 25 is a diagram showing an operator and the coordinate system of the operation unit.
FIG. 26 is a diagram showing buttons displayed on a touch panel to change a predetermined angle.
FIG. 27 is a flowchart for illustrating a control method for the robotic surgical system according to the embodiment.
FIG. 28 is a diagram for illustrating setting of a virtual plane.

### DESCRIPTION OF THE PREFERRED EMBODIMENTS

### Configuration of Robotic Surgical System

The configuration of a robotic surgical system 500 according to this embodiment is now described. The robotic surgical system 500 includes a surgical robot 100, a remote control apparatus 200, a vision unit 300, and an image processing unit 400. The surgical robot 100 is an example of a surgical apparatus, and the remote control apparatus 200 is an example of an operation apparatus.

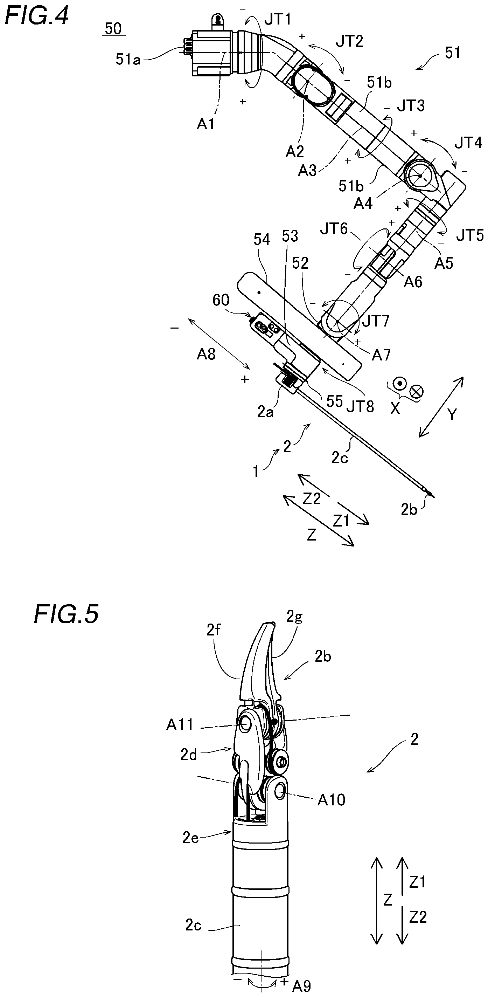

In this specification, as shown in FIG. 4, the longitudinal direction of a surgical instrument 1 is defined as a Z direction. The distal end side of the surgical instrument 1 is defined as a Z1 side, and the proximal end side of the surgical instrument 1 is defined as a Z2 side. A direction perpendicular to the Z direction is defined as an X direction. A direction perpendicular to the Z direction and the X direction is defined as a Y direction.

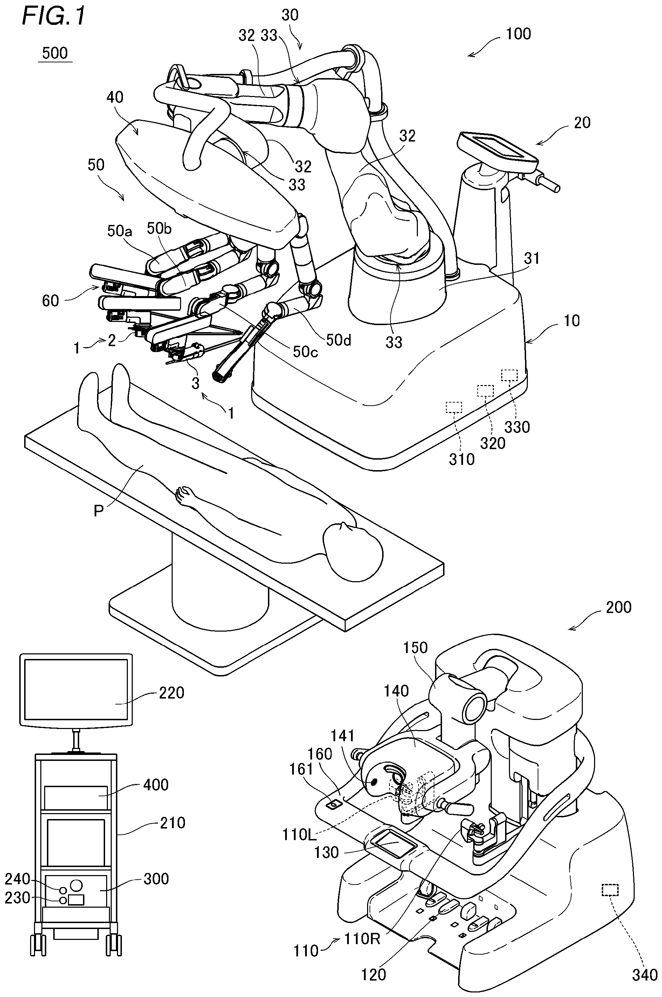

As shown in FIG. 1, the surgical robot 100 is arranged in an operating room. The remote control apparatus 200 is spaced apart from the surgical robot 100. The remote control apparatus 200 receives operations for the surgical instrument 1. Specifically, an operator such as a doctor inputs a command to the remote control apparatus 200 to cause the surgical robot 100 to perform a desired operation. The remote control apparatus 200 transmits the input command to the surgical robot 100. The surgical robot 100 operates based on the received command. The surgical robot 100 is arranged in the operating room that is a sterilized sterile field.

### Configuration of Surgical Robot

As shown in FIG. 1, the surgical robot 100 includes a medical cart 10, a cart positioner operation unit 20, a positioner 30, an arm base 40, a plurality of robot arms 50, and an arm operation unit 60 provided on each of the robot arms 50.

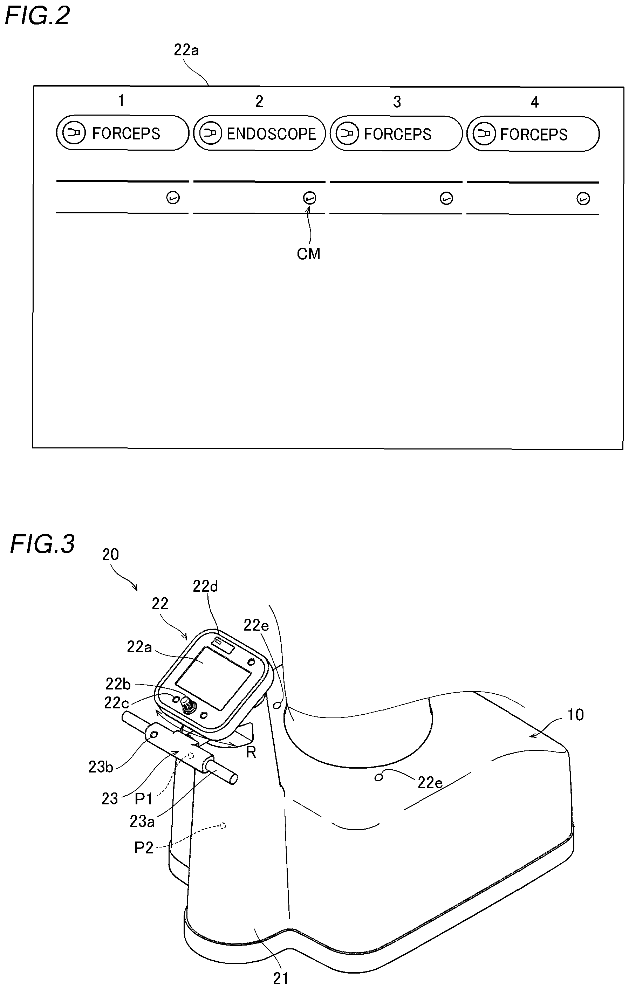

As shown in FIG. 3, the cart positioner operation unit 20 is supported by a cart positioner operation support 21 at the rear of the medical cart 10, and the medical cart 10 or the positioner 30 is moved by operating the cart positioner operation unit 20. The cart positioner operation unit 20 includes an input 22 and an operation handle 23. The input 22 receives operations to move the positioner 30, the arm base 40, and the plurality of robot arms 50 or change their postures mainly in order to prepare for surgery before the surgery. The medical cart 10 includes the operation handle 23.

As shown in FIG. 3, the input 22 of the medical cart 10 includes a display 22a, a joystick 22b, an enable switch 22c, an error reset button 22d, and speakers 22e. The display 22a is a liquid crystal panel, for example. As shown in FIG. 2, the display 22a displays numbers corresponding to the plurality of robot arms 50. The display 22a also displays the type of surgical instrument 1 attached to each of the plurality of robot arms 50. A check mark CM indicating that a pivot position PP described below has been set is displayed on the display 22a.

As shown in FIG. 3, the joystick 22b is arranged in the vicinity of or adjacent to the display 22a of the input 22 of the medical cart 10. The positioner 30 is moved three-dimensionally by selecting an operation mode displayed on the display 22a and operating the joystick 22b.

The enable switch 22c is arranged in the vicinity of or adjacent to the joystick 22b. The enable switch 22c enables or disables movement of the positioner 30. When the joystick 22b is operated while the enable switch 22c is pressed to enable movement of the positioner 30, the positioner 30 is moved.

The error reset button 22d resets errors in the robotic surgical system 500. The errors may be abnormal deviation errors, for example. The speakers 22e are arranged in pair. The pair of speakers 22e are arranged in the vicinity of or adjacent to the location of the positioner 30 in the medical cart 10.

The operation handle 23 is arranged in the vicinity of the display 22a. The operation handle 23 includes a throttle 23a that is gripped and twisted by an operator such as a nurse or a technician to operate movement of the medical cart 10. Specifically, the operation handle 23 is arranged below the input 22. As the throttle 23a is twisted from the near side to the far side, the medical cart 10 moves forward. As the throttle 23a is twisted from the far side to the near side, the medical cart 10 moves rearward. The speed of the medical cart 10 is changed according to a twisting amount of the throttle 23a. The operation handle 23 is rotatable to the left and right shown by an R direction, and the medical cart 10 is turned with rotation of the operation handle 23.

An enable switch 23b for enabling or disabling movement of the medical cart 10 is provided on the operation handle 23 of the cart positioner operation unit 20. When the throttle 23a of the operation handle 23 is operated while the enable switch 23b is pressed to enable movement of the medical cart 10, the medical cart 10 is moved.

As shown in FIG. 1, the positioner 30 includes a 7-axis articulated robot, for example. The positioner 30 is arranged on the medical cart 10. The positioner 30 adjusts the position of the arm base 40. The positioner 30 moves the position of the arm base 40 three-dimensionally.

The positioner 30 includes a base 31 and a plurality of links 32 coupled to the base 31. The plurality of links 32 are coupled to each other by joints 33.

The arm base 40 is attached to the distal end of the positioner 30. The proximal end of each of the plurality of robot arms 50 is attached to the arm base 40. Each of the plurality of robot arms 50 is able to take a folded and stored posture. The arm base 40 and the plurality of robot arms 50 are covered with sterile drapes and used. Moreover, each of the robot arms 50 supports the surgical instrument 1.

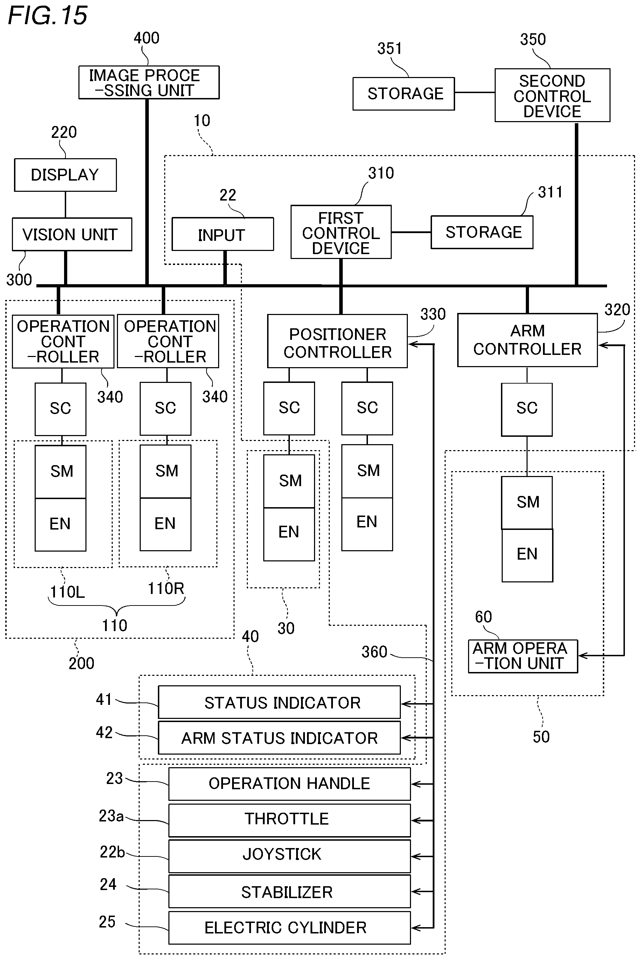

A status indicator 41 and an arm status indicator 42 that are shown in FIG. 15 are provided on the arm base 40. The status indicator 41 indicates the status of the robotic surgical system 500. The arm status indicator 42 indicates the statuses of the robot arms 50.

The plurality of robot arms 50 are arranged. Specifically, four robot arms 50a, 50b, 50c, and 50d are arranged. The robot arms 50a, 50b, 50c, and 50d have the same or similar configurations as each other.

As shown in FIG. 4, each robot arm 50 includes an arm portion 51, a first link 52, a second link 53, and a translation mechanism 54. The robot arm 50 includes joints JT1, JT2, JT3, JT4, JT5, JT6, JT7, and JT8. The joints JT1, JT2, JT3, JT4, JT5, JT6, and JT7 have A1, A2, A3, A4, A5, A6, and A7 axes as rotation axes, respectively. The joint JT8 has an A8 axis as a linear motion axis. The arm portion 51 includes a base 51a and links 51b.

The arm portion 51 includes a 7-axis articulated robot arm. The first link 52 is arranged at the distal end of the arm portion 51. The arm operation unit 60 described below is attached to the second link 53. The translation mechanism 54 is arranged between the first link 52 and the second link 53. A holder 55 that holds the surgical instrument 1 is arranged on the second link 53. The translation mechanism 54 translates the holder 55 to which the surgical instrument 1 is attached between a first position and a second position. The first position refers to an end position on the Z2 side in a range of movement of the holder 55 along the A8 axis by the translation mechanism 54. The second position refers to an end position on the Z1 side in the range of movement of the holder 55 along the A8 axis by the translation mechanism 54.

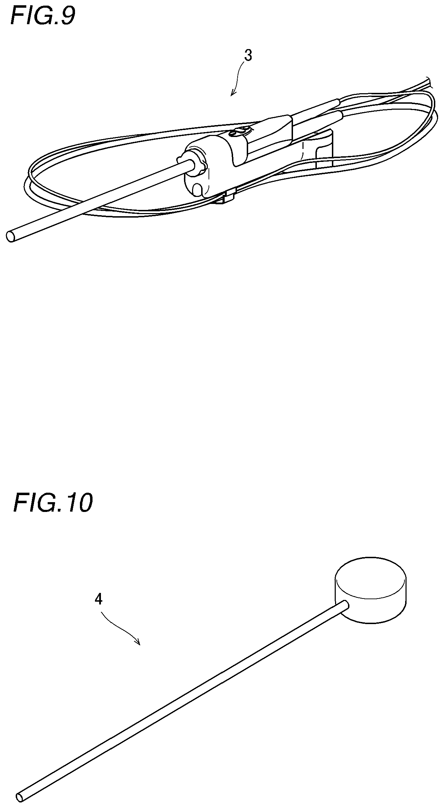

The surgical instrument 1 is attached to the distal end of each of the plurality of robot arms 50. The surgical instrument 1 includes a replaceable instrument 2, an endoscope 3 shown in FIG. 9 to capture an image of a surgical site, and a pivot position setting instrument 4 shown in FIG. 10 to set the pivot position PP, for example. The instrument 2 includes a driven unit 2a, a pair of forceps 2b, and a shaft 2c. The instrument 2 and the endoscope 3 are examples of a surgical instrument.

As shown in FIG. 1, the endoscope 3 is attached to the distal end of one of the plurality of robot arms 50, such as the robot arm 50c, and the instrument 2 is attached to the distal end of each of the remaining robot arms 50a, 50b, and 50d, for example. The endoscope 3 is preferably attached to one of two robot arms 50b and 50c arranged in the center among the four robot arms 50 arranged adjacent to each other.

### Configuration of Instrument

As shown in FIG. 5, the pair of forceps 2b is attached to the distal end of the instrument 2, for example. At the distal end of the instrument 2, in addition to the pair of forceps 2b, a pair of scissors, a grasper, a needle holder, a microdissector, a stable applier, a tacker, a suction cleaning tool, a snare wire, a clip applier, etc. are arranged as instruments having joints. At the distal end of the instrument 2, a cutting blade, a cautery probe, a washer, a catheter, a suction orifice, etc. are arranged as instruments having no joint.

The pair of forceps 2b includes a first support 2d and a second support 2e. The first support 2d supports the proximal end sides of jaw members 2f and 2g such that the jaw members 2f and 2g are rotatable about an A11 axis. The second support 2e supports the proximal end side of the first support 2d such that the first support 2d is rotatable about an A10 axis. The shaft 2c rotates about an A9 axis. The jaw members 2f and 2g pivot about the A11 axis to open and close.

### Configuration of Arm Operation Unit

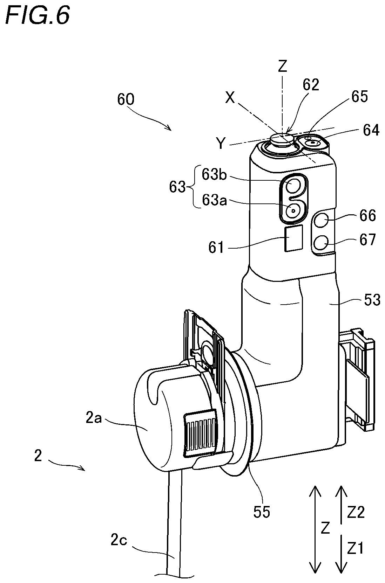

As shown in FIG. 6, the arm operation unit 60 is attached to the robot arm 50 to operate the robot arm 50. Specifically, the arm operation unit 60 is attached to the second link 53.

The arm operation unit 60 includes an enable switch 61, a joystick 62, and linear switches 63, a mode switching button 64, a mode indicator 65, a pivot button 66, and an adjustment button 67.

The enable switch 61 is pressed to enable or disable movement of the robot arm 50 in response to the joystick 62 and the linear switches 63. When the enable switch 61 is pressed by an operator such as a nurse or an assistant grasping the arm operation unit 60, movement of the surgical instrument 1 by the robot arm 50 is enabled.

The joystick 62 is an operation tool to control movement of the surgical instrument 1 by the robot arm 50. The joystick 62 controls a moving direction and a moving speed of the robot arm 50. The robot arm 50 is moved in accordance with a tilting direction and a tilting angle of the joystick 62.

The linear switches 63 are switches to move the surgical instrument 1 in the Z direction, which is the longitudinal direction of the surgical instrument 1. The linear switches 63 include a linear switch 63a to move the surgical instrument 1 in a direction in which the surgical instrument 1 is inserted into a patient P, and a linear switch 63b to move the surgical instrument 1 in a direction in which the surgical instrument 1 is moved away from the patient P. Both the linear switch 63a and the linear switch 63b are push-button switches.

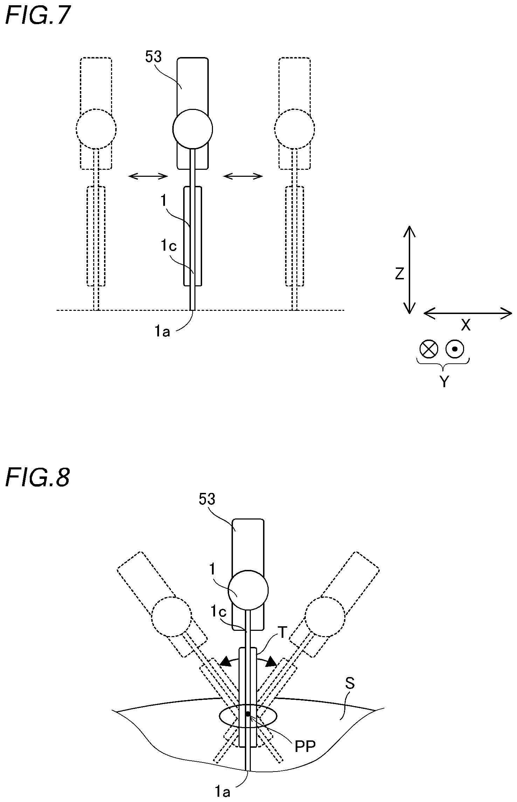

The mode switching button 64 is a push-button switch to switch between a mode for translationally moving the surgical instrument 1 and a mode for rotationally moving the surgical instrument 1. As shown in FIG. 7, in the mode for translationally moving the robot arm 50, the robot arm 50 is moved such that the distal end 1a of the surgical instrument 1 is moved in an X-Y plane. As shown in FIG. 8, in the mode for rotationally moving the robot arm 50, the robot arm 50 is moved such that the surgical instrument 1 is rotationally moved about a center on the A11 axis of the pair of forceps 2b or the distal end of the pair of forceps 2b of the instrument 2 as the surgical instrument 1 as a fulcrum when any pivot position PP is not stored in a storage 351, and the surgical instrument 1 is rotationally moved about the pivot position PP as a fulcrum when the pivot position PP is stored in the storage 351. In this case, the surgical instrument 1 is rotationally moved with the shaft 1c of the surgical instrument 1 inserted into a trocar T. The mode switching button 64 is arranged on a Z-direction side surface of the arm operation unit 60.

The mode indicator 65 indicates a switched mode. The mode indicator 65 is on to indicate a rotational movement mode and is off to indicate a translational movement mode. Furthermore, the mode indicator 65 also serves as a pivot position indicator that indicates that the pivot position PP has been set. The mode indicator 65 is arranged on the Z-direction side surface of the arm operation unit 60.

The pivot button 66 is a push-button switch to set the pivot position PP that serves as a fulcrum for movement of the surgical instrument 1 attached to the robot arm 50.

The adjustment button 67 is a button to optimize the position of the robot arm 50. After the pivot position PP for the robot arm 50 to which the endoscope 3 has been attached is set, the positions of the other robot arms 50 and the arm base 40 are optimized when the adjustment button 67 is pressed. The adjustment button 67 is a button different from the enable switch 61.

### Remote Control Apparatus

The remote control apparatus 200 receives operations for the surgical instrument 1. As shown in FIG. 1, the remote control apparatus 200 is arranged inside or outside the operating room, for example. The remote control apparatus 200 includes the operation units 110, foot pedals 120, a touch panel 130, a monitor 140, a support arm 150, a support bar 160, an error reset button 161. The operation units 110 include operation handles for the operator such as a doctor to input a command. The monitor 140 is an example of a display.

### Operation Unit

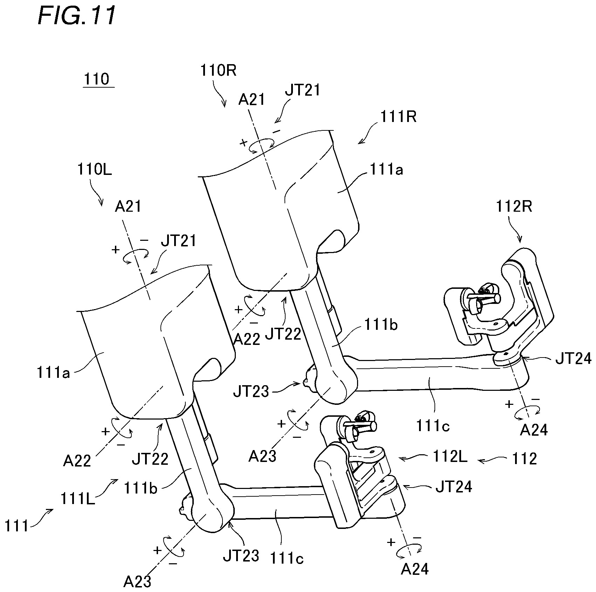

As shown in FIG. 11, the operation units 110 each include a handle to operate the surgical instrument 1. The operation unit 110 receives an operation for the surgical instrument 1. The operation units 110 include an operation unit 110L that is located on the left side as viewed from the operator such as a doctor and is to be operated by the left hand of the operator, and an operation unit 110R that is located on the right side and is to be operated by the right hand of the operator. The operation units 110 include arms 111 and wrists 112. The operation unit 110R includes an arm 111R and a wrist 112R. The operation unit 110L includes an arm 111L and a wrist 112L.

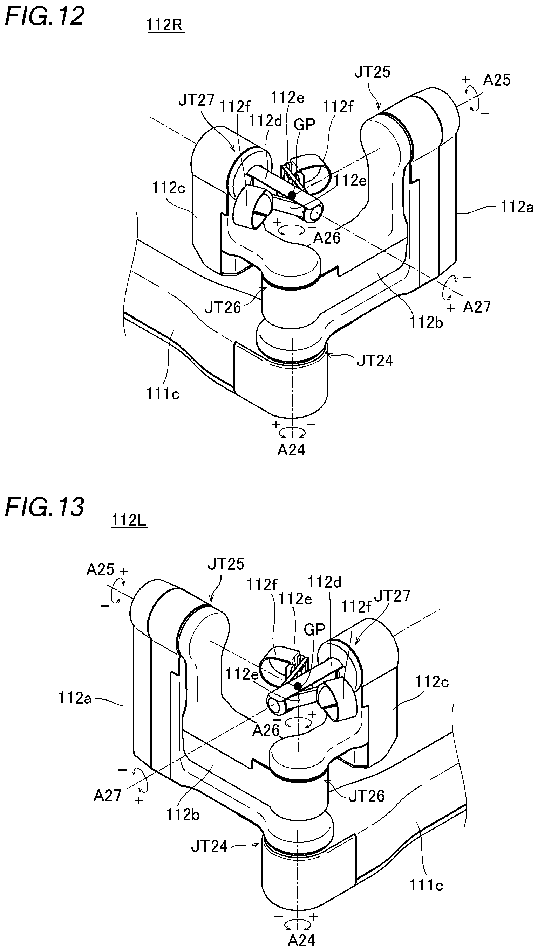

The operation units 110 each have joints JT21, JT22, and JT23 shown in FIG. 11, and joints JT24, JT25, JT26, and JT27 shown in FIGS. 12 and 13. The rotation axes of the joints JT21, JT22, JT23, JT24, JT25, JT26, and JT27 are A21, A22, A23, A24, A25, A26, and A27 axes, respectively.

As shown in FIG. 11, the arm 111R includes a link 111a, a link 111b, and a link 111c. The upper end side of the link 111a is attached to the remote control apparatus 200 such that the link 111a is rotatable about the A21 axis along a vertical direction. The upper end side of the link 111b is attached to the lower end side of the link 111a such that the link 111b is rotatable about the A22 axis along a horizontal direction. A first end side of the link 111c is attached to the lower end side of the link 111b such that the link 111c is rotatable about the A23 axis along the horizontal direction. The wrist 112 is attached to a second end side of the link 111c such that the wrist 112 is rotatable about the A24 axis. The link 111a is connected to the remote control apparatus 200 by the joint JT21. The link 111a and the link 111b are connected to each other by the joint JT22. The link 111b and the link 111c are connected to each other by the joint JT23. The arm 111 supports the wrist 112. The arm 111L has the same or similar configuration as the arm 111R.

The wrists 112 include the wrist 112R shown in FIG. 12 and to be operated by the right hand of the operator, and the wrist 112L shown in FIG. 13 and to be operated by the left hand of the operator. FIG. 12 shows the reference posture of the operation unit 110R, and FIG. 13 shows the reference posture of the operation unit 110L. The configuration of the wrist 112R is the same as or similar to that of the wrist 112L.

The wrists 112 each include a link 112a, a link 112b, a link 112c, and a grip 112d that is to be operated by the operator such as a doctor. The link 112a includes a proximal end connected to the distal end of the arm 111 and rotates about the A24 axis. The link 112b includes a proximal end connected to the distal end of the link 112a and rotates about the A25 axis with respect to the link 112a. The link 112c includes a proximal end connected to the distal end of the link 112b and a distal end to which the grip 112d is connected, and rotates about the A26 axis with respect to the link 112b. The grip 112d rotates about the A27 axis with respect to the link 112c. The link 112a, the link 112b, and the link 112c each have an L shape.

The wrist 112 includes a pair of grip members 112e that are opened and closed by the operator. The grip members 112e each include an elongated plate-shaped lever member, and the proximal ends of the pair of grip members 112e are rotatably connected to the proximal end of the grip 112d. Cylindrical finger insertion portions 112f are provided on the grip members 112e. The operator inserts their fingers into a pair of finger insertion portions 112f to operate the wrist 112. The proximal ends of the pair of grip members 112e are connected to the grip 112d, and an angle between the pair of grip members 112e is increased or decreased such that an opening angle between the jaw member 2f and the jaw member 2g is changed. A magnet is provided on one of the grip members 112e, and a Hall sensor is provided on the grip 112d. When the operator opens and closes the grip members 112e, the magnet and the Hall sensor function as an angle detection sensor, and the Hall sensor outputs the opening angle. As the angle detection sensor, the Hall sensor may be provided on the grip member 112e, and the magnet may be provided on the grip 112d. Alternatively, the magnet or the Hall sensor may be provided as the angle detection sensor on both the grip members 112e.

As shown in FIG. 1, the monitor 140 is a scope-type display that displays images captured by the endoscope 3. A notifier 141 is provided on the monitor 140. The notifier 141 issues an error sound. The support arm 150 supports the monitor 140 so as to align the height of the monitor 140 with the height of the face of the operator such as a doctor. The touch panel 130 is arranged on the support bar 160. The touch panel 130 receives settings for the robotic surgical system 500. The head of the operator is detected by a sensor provided in the vicinity of the monitor 140 such that the surgical robot 100 can be operated by the remote control apparatus 200. The operator operates the operation units 110 and the foot pedals 120 while visually recognizing an affected area on the monitor 140. Thus, a command is input to the remote control apparatus 200. The command input to the remote control apparatus 200 is transmitted to the surgical robot 100.

The error reset button 161 is arranged on the support bar 160. The error reset button 161 resets errors in the robotic surgical system 500. The errors may be abnormal deviation errors, for example.

### Foot Pedals

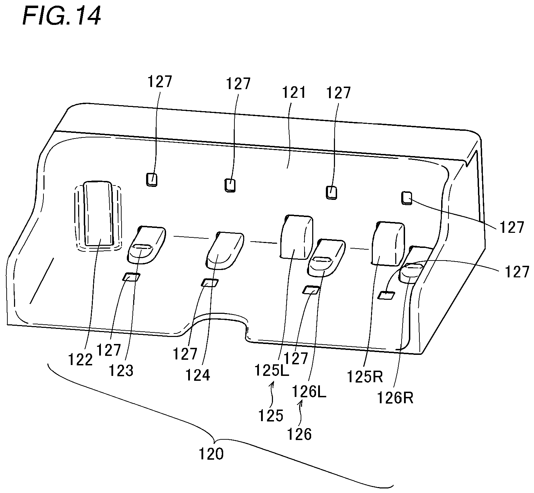

As shown in FIG. 14, a plurality of foot pedals 120 are provided to perform functions related to the surgical instrument 1. The plurality of foot pedals 120 are arranged on a base 121. The foot pedals 120 include a switching pedal 122, a clutch pedal 123, a camera pedal 124, incision pedals 125, coagulation pedals 126, and foot detectors 127. The switching pedal 122, the clutch pedal 123, the camera pedal 124, the incision pedals 125, and the coagulation pedals 126 are operated by the foot of the operator. The incision pedals 125 include an incision pedal 125R for a right robot arm 50, and an incision pedal 125L for a left robot arm 50. The coagulation pedals 126 include a coagulation pedal 126R for the right robot arm 50 and a coagulation pedal 126L for the left robot arm 50.

The switching pedal 122 switches robot arms 50 to be operated by the operation units 110. The clutch pedal 123 performs a clutch operation to temporarily disconnect an operation connection between the robot arms 50 and the operation units 110. While the clutch pedal 123 is being pressed by the operator, operations by the operation units 110 are not transmitted to the robot arms 50. While the camera pedal 124 is being pressed by the operator, the operation unit 110 can operate a robot arm 50 to which the endoscope 3 is attached. While the incision pedals 125 or the coagulation pedals 126 are being pressed by the operator, an electrosurgical device is activated.

The foot detectors 127 detect the foot of the operator that operates the foot pedals 120. The foot detectors 127 are provided for the switching pedal 122, the clutch pedal 123, the camera pedal 124, the incision pedal 125L, the coagulation pedal 126L, the incision pedal 125R, and the coagulation pedal 126R, and detect the foot that hovers above their corresponding foot pedals 120. The foot detectors 127 are arranged on the base 121. The functions of the foot pedals 120 including the camera pedal 124 are not limited to being performed through pedals configured to be pressed by the foot of the operator as in this embodiment, but inputs such as hand switches may be provided on the operation units 110, and manual operations by the operator may be used, for example.

### Vision Unit and Image Processing Unit

As shown in FIG. 1, the vision unit 300 and the image processing unit 400 are placed on a cart 210. The image processing unit 400 processes images captured by the endoscope 3. A display 220 is arranged on the cart 210. The display 220 displays images captured by the endoscope 3. An error reset button 230 and a notifier 240 are arranged on the vision unit 300. The error reset button 230 resets errors in the robotic surgical system 500. The errors may be abnormal deviation errors, for example. The notifier 240 issues an error sound.

### Configuration of Control System

As shown in FIG. 15, the robotic surgical system 500 includes a first control device 310, an arm controller 320, a positioner controller 330, operation controllers 340, and a second control device 350. The robotic surgical system 500 also includes a storage 311 connected to the first control device 310 and the storage 351 connected to the second control device 350. The first control device 310 is an example of a controller. The second control device 350 is an example of a monitoring controller.

The first control device 310 is accommodated in the medical cart 10 to communicate with the arm controller 320 and the positioner controller 330, and controls the entire robotic surgical system 500. Specifically, the first control device 310 communicates with and controls the arm controller 320, the positioner controller 330, and the operation controllers 340. The first control device 310 is connected to the arm controller 320, the positioner controller 330, and the operation controllers 340 through a LAN, for example. The first control device 310 is arranged inside the medical cart 10.

The arm controller 320 is arranged for each of the plurality of robot arms 50. That is, the same number of arm controllers 320 as the plurality of robot arms 50 are placed inside the medical cart 10.

As shown in FIG. 15, the input 22 is connected to the first control device 310 through a LAN, for example. The status indicator 41, the arm status indicator 42, the operation handle 23, the throttle 23a, and the joystick 22b are connected to the positioner controller 330 via a wire line 360 by means of a communication network that allows information to be shared with each other by using serial communication. Although FIG. 15 shows that the status indicator 41, the arm status indicator 42, etc. are all connected to one wire line 360, in reality, the wire line 360 is arranged for each of the status indicator 41, the arm status indicator 42, the operation handle 23, the throttle 23a, and the joystick 22b.

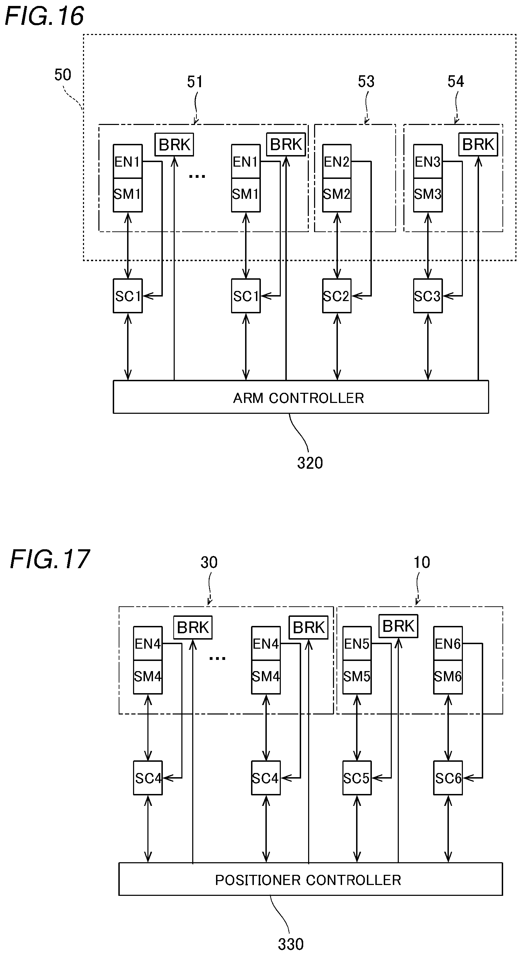

As shown in FIG. 16, the arm portion 51 includes a plurality of servomotors SM1, encoders EN1, and speed reducers so as to correspond to the joints JT1, JT2, JT3, JT4, JT5, JT6, and JT7. The encoders EN1 detect rotation angles of the servomotors SM1. The speed reducers slow down rotation of the servomotors SM1 to increase the torques. Inside the medical cart 10, servo controllers SC1 that control the servomotors SM1 are arranged adjacent to the arm controller 320. In addition, the encoders EN1 that detect the rotation angles of the servomotors SM1 are electrically connected to the servo controllers SC1.

Servomotors SM2 that rotate driven members provided in the driven unit 2a of the instrument 2, encoders EN2, and speed reducers are arranged in the second link 53. The encoders EN2 detect rotation angles of the servomotors SM2. The speed reducers slow down rotation of the servomotors SM2 to increase the torques. In the medical cart 10, servo controllers SC2 are provided to control the servomotors SM2 to drive the surgical instrument 1. The encoders EN2 that detect the rotation angles of the servomotors SM2 are electrically connected to the servo controllers SC2. A plurality of servomotors SM2, a plurality of encoders EN2, and a plurality of servo controllers SC2 are arranged.

The translation mechanism 54 includes a servomotor SM3 to translationally move the surgical instrument 1, an encoder EN3, and a speed reducer. The encoder EN3 detects a rotation angle of the servomotor SM3. The speed reducer slows down rotation of the servomotor SM3 to increase the torque. In the medical cart 10, a servo controller SC3 is provided to control the servomotor SM3 to translationally move the surgical instrument 1. The encoder EN3 that detects the rotation angle of the servomotor SM3 is electrically connected to the servo controller SC3.

The first control device 310 generates command values to command the positions of the servomotors SM1, SM2, and SM3 based on operations received by the remote control apparatus 200, and drives the servomotors SM1, SM2, and SM3 based on the command values. The first control device 310 detects an abnormal deviation error when differences between the command values and the positions of the servomotors SM1, SM2, and SM3 detected by sensors exceed an allowable range.

As shown in FIG. 17, a plurality of servomotors SM4, a plurality of encoders EN4, and a plurality of speed reducers are provided in the positioner 30 so as to correspond to a plurality of joints 33 of the positioner 30. The encoders EN4 detect rotation angles of the servomotors SM4. The speed reducers slow down rotation of the servomotors SM4 to increase the torques.

The medical cart 10 includes wheels including front wheels as drive wheels and rear wheels that are steered by the operation handle 23. The rear wheels are arranged closer to the operation handle 23 than the front wheels. The medical cart 10 includes servomotors SM5 to drive a plurality of front wheels of the medical cart 10, encoders EN5, speed reducers, and brakes BRK. The speed reducers slow down rotation of the servomotors SM5 to increase the torques. A potentiometer P1 shown in FIG. 3 is provided on the operation handle 23 of the medical cart 10, and the servomotors SM5 of the front wheels are driven based on a rotation angle detected by the potentiometer P1 according to the twist of the throttle 23a. Rear wheels of the medical cart 10 are of the dual wheel type, and the rear wheels are steered based on rightward-leftward rotation of the operation handle 23. Furthermore, a potentiometer P2 shown in FIG. 3 is provided on a rotation axis of the operation handle 23 of the medical cart 10, and servomotors SM6, encoders EN6, and speed reducers are provided on the rear wheels of the medical cart 10. The speed reducers slow down rotation of the servomotors SM6 to increase the torques. The servomotors SM6 are driven based on a rotation angle detected by the potentiometer P2 according to rightward-leftward rotation of the operation handle 23. That is, steering of the rear wheels by the rightward-leftward rotation of the operation handle 23 is power-assisted by the servomotors SM6.

The front wheels of the medical cart 10 are driven such that the medical cart 10 moves in a forward-rearward direction. Furthermore, the operation handle 23 of the medical cart 10 is rotated such that the rear wheels are steered, and the medical cart 10 turns in a right-left direction.

As shown in FIG. 17, in the medical cart 10, servo controllers SC4 are provided to control the servomotors SM4 to move the positioner 30. The encoders EN4 that detect the rotation angles of the servomotors SM4 are electrically connected to the servo controllers SC4. In the medical cart 10, servo controllers SC5 are provided to control the servomotors SM5 to drive the front wheels of the medical cart 10. The encoders EN5 that detect the rotation angles of the servomotors SM5 are electrically connected to the servo controllers SC5. In the medical cart 10, servo controllers SC6 are provided to control the servomotors SM6 to power-assist steering of the rear wheels of the medical cart 10. The encoders EN6 that detect the rotation angles of the servomotors SM6 are electrically connected to the servo controllers SC6.

As shown in FIGS. 16 and 17, the joints JT1, JT2, JT3, JT4, JT5, JT6, and JT7 of the arm portion 51, and the joints 33 of the positioner 30 include brakes BRK. Furthermore, the front wheels of the medical cart 10, the arm base 40, and the translation mechanism 54 include brakes BRK. The arm controller 320 unidirectionally transmits control signals to the brakes BRK of the joints JT1, JT2, JT3, JT4, JT5, JT6, and JT7 of the arm portion 51, and the translation mechanism 54. The control signals are signals for turning on/off the brakes BRK. The signals for turning on the brakes BRK include signals for maintaining the brakes BRK in an enabled state. The same applies to control signals from the positioner controller 330 to the brakes BRK of the joints 33 of the positioner 30 and the arm base 40. On startup, all the brakes BRK of the arm base 40, the arm portion 51, and the translation mechanism 54 are turned off but the servomotors SM are driven against gravity to maintain the postures of the robot arm 50 and the arm base 40. When an error occurs in the robotic surgical system 500, the brakes BRK of the arm base 40, the arm portion 51 and the translation mechanism 54 are turned on. When the error in the robotic surgical system 500 is reset, the brakes BRK of the arm base 40, the arm portion 51, and the translation mechanism 54 are turned off. When a shutdown operation is performed in the robotic surgical system 500, the brakes BRK of the arm base 40, the arm portion 51, and the translation mechanism 54 are turned on. The brakes BRK of the front wheels of the medical cart 10 are constantly turned on, and the brakes BRK are deactivated only while the enable switch 23b of the medical cart 10 is being pressed. The brakes BRK of the joints 33 of the positioner 30 are constantly turned on, and the brakes BRK are deactivated only while the enable switch 22c of the medical cart 10 is being pressed.

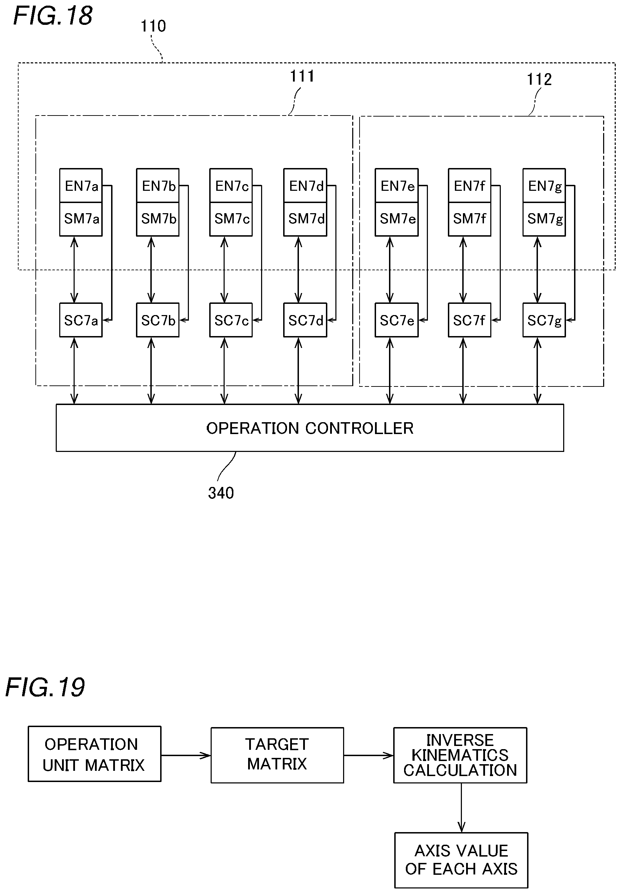

As shown in FIG. 18, servomotors SM7a, SM7b, SM7c, SM7d, SM7e, SM7f, and SM7g are arranged on the joints JT21, JT22, JT23, JT24, JT25, JT26, and JT27 of the operation unit 110, respectively. The servomotor SM7a rotates the link 111a about the A21 axis. The servomotor SM7b rotates the link 111b about the A22 axis. The servomotor SM7c rotates the link 111c about the A23 axis. The servomotor SM7d rotates the link 112a about the A24 axis. The servomotor SM7e rotates the link 112b about the A25 axis. The servomotor SM7f rotates the link 112c about the A26 axis. The servomotor SM7g rotates the grip 112d about the A27 axis. Servo controllers SC7a, SC7b, SC7c, SC7d, SC7e, SC7f, and SC7g are provided to control the servomotors. Encoders EN7a, EN7b, EN7c, EN7d, EN7e, EN7f, and EN7g are electrically connected to the servo controllers to detect rotation angles of the servomotors. The servomotors, the servo controllers, and the encoders are provided in each of the operation unit 110L and the operation unit 110R.

The first control device 310 controls the servomotors via the operation controllers 340 to generate torques that counteract gravitational torques generated on rotation axes of the servomotors according to the postures of the operation units 110. Thus, the operator can operate the operation units 110 with a relatively small force.

As shown in FIG. 15, the first control device 310 controls the robot arm 50 based on an operation received by the arm operation unit 60. For example, the first control device 310 controls the robot arm 50 based on an operation received by the joystick 62 of the arm operation unit 60. Specifically, the arm controller 320 outputs an input signal input from the joystick 62 to the first control device 310. The first control device 310 generates position commands based on the received input signal and the rotation angles detected by the encoders EN1, and outputs the position commands to the servo controllers SC1 via the arm controller 320. The servo controllers SC1 generate current commands based on the position commands input from the arm controller 320 and the rotation angles detected by the encoders EN1, and output the current commands to the servomotors SM1. Thus, the robot arm 50 is moved according to an operation command input to the joystick 62.

The first control device 310 controls the robot arm 50 based on an input signal from either linear switch 63 of the arm operation unit 60. Specifically, the arm controller 320 outputs the input signal input from the linear switch 63 to the first control device 310. The first control device 310 generates a position command(s) based on the received input signal and the rotation angle(s) detected by the encoders EN1 or the encoder EN3, and outputs the position command(s) to the servo controllers SC1 or the servo controller SC3 via the arm controller 320. The servo controllers SC1 or the servo controller SC3 generates a current command(s) based on the position command(s) input from the arm controller 320 and the rotation angle(s) detected by the encoders EN1 or the encoder EN3, and outputs the current command(s) to the servomotors SM1 or the servomotor SM3. Thus, the robot arm 50 is moved according to an operation command input to the linear switch 63.

The positioner controller 330 is arranged in the medical cart 10. The positioner controller 330 controls the positioner 30 and the medical cart 10. The servomotors SM4, the encoders EN4, and the speed reducers are provided in the positioner 30 so as to correspond to the plurality of joints 33 of the positioner 30. The servo controllers SC4 are provided in the medical cart 10 to control the servomotors SM4 of the positioner 30. The servomotors SM5 and SM6 that drive the plurality of front wheels of the medical cart 10, the encoders EN5 and EN6, the speed reducers, the servo controllers SC5 and SC6, and the brakes BRK are provided in the medical cart 10.

The operation controllers 340 are arranged in a main body of the remote control apparatus 200. The operation controllers 340 control the operation units 110. The operation controllers 340 are provided so as to correspond to the left-handed operation unit 110L and the right-handed operation unit 110R, respectively, as shown in FIG. 15.

As shown in FIG. 15, the vision unit 300 and the image processing unit 400 are connected to the first control device 310 through a LAN, for example. The display 220 is connected to the vision unit 300.

### Control Operation of First Control Device

A control performed by the first control device 310 when the operation unit 110 receives an operation by the operator is now described.

As shown in FIG. 19, the first control device 310 presets an operation unit matrix for the operation unit 110. The operation unit matrix is a homogeneous transformation matrix of a 4 × 4 matrix. The operation unit matrix represents the result of forward kinematics calculation for the position and posture of the operation unit 110. The operation unit matrix is set based on the coordinate system C1 of the operation unit 110. The coordinate system C1 of the operation unit 110 is described below. Next, the operation by the operator is received by the operation unit 110. Thus, a target matrix that is a target corresponding to the received operation is generated. The target matrix is also a homogeneous transformation matrix. The target matrix represents target values of the position and posture of the surgical instrument 1. The target matrix is set based on the coordinate system of the surgical robot 100. The homogeneous transformation matrix includes a translation component for translation of the surgical instrument 1 and a rotation component for rotation of the surgical instrument 1. Specifically, the first control device 310 calculates a difference between the current position of the surgical instrument 1 and a target position received by the operation unit 110. The position corresponds to the translation component of the homogeneous transformation matrix. The first control device 310 calculates a difference between the current posture of the surgical instrument 1 and a target posture received by the operation unit 110. The posture corresponds to the rotation component of the homogeneous transformation matrix. The first control device 310 calculates the target matrix based on the calculated difference value. Next, the first control device 310 performs an inverse kinematics calculation on the updated homogeneous transformation matrix. The first control device 310 calculates axis values of joint axes and linear motion axes for the robot arm 50 and the surgical instrument 1 by the inverse kinematics calculation. Thus, the positions and postures of the robot arm 50 and the surgical instrument 1 are changed to conform to the received operation.

### Coordinate Systems of Robotic Surgical System

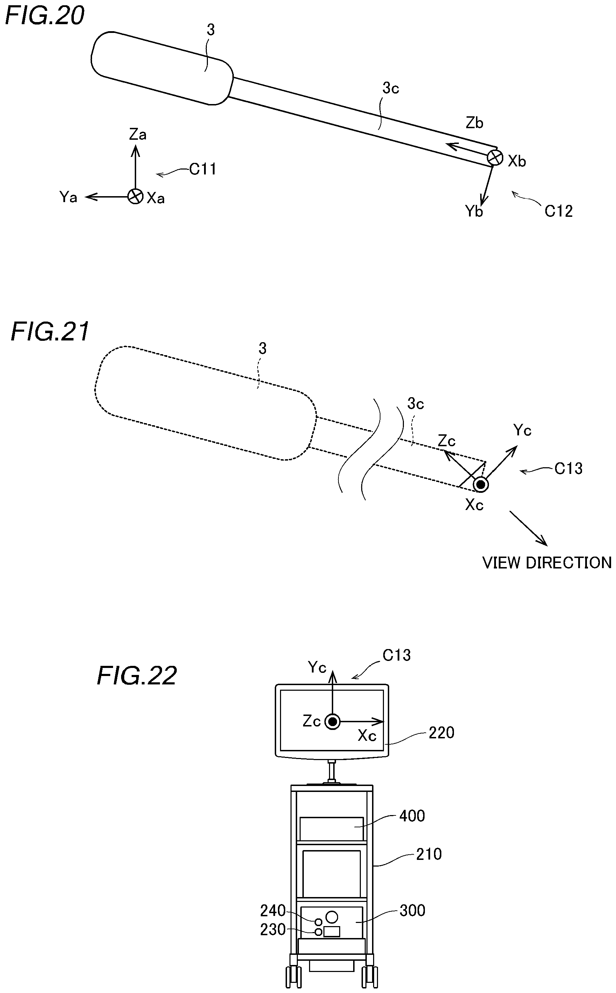

The coordinate systems set in the robotic surgical system 500 are now described. First, the coordinate system of the surgical robot 100 is described. As shown in FIGS. 20 and 21, the coordinate system of the surgical robot 100 includes a base coordinate system C11, a tool coordinate system C12, and an endoscope coordinate system C13. The three axes of the base coordinate system C11 are defined as an Xa axis, a Ya axis, and a Za axis. The three axes of the tool coordinate system C12 are defined as an Xb axis, a Yb axis, and a Zb axis. The three axes of the endoscope coordinate system C13 are defined as an Xc axis, a Yc axis, and a Zc axis. The origin of the base coordinate system C11 is an intersection of the rotation axis of the proximal end of the positioner 30 with the mounting surface of the medical cart 10 mounted on the positioner 30. The origin of the tool coordinate system C12 is set to the clevis position of the pair of forceps 2b when the surgical instrument 1 is an instrument 2, and is set to the distal end position of the endoscope 3 when the surgical instrument 1 is an endoscope 3. The Zb axis of the tool coordinate system C12 is along a direction in which the shaft 2c of the instrument 2 or a shaft 3c of the endoscope 3 extends. The endoscope coordinate system C13 is obtained by rotating the tool coordinate system C12 by 180 degrees around the Zb axis. When the surgical instrument 1 is an endoscope 3, as shown in FIG. 21, a direction of view of the endoscope 3 may intersect with the direction in which the shaft 3c of the endoscope 3 extends. In such a case, the endoscope coordinate system C13 is obtained by rotating the tool coordinate system C12 by 180 degrees around the Zb axis and further rotating it around the Xb axis by the intersection angle of the direction of view of the endoscope 3.

In this embodiment, an image captured by the endoscope 3 is displayed on the display 220 of the cart 210 shown in FIG. 22. The image displayed on the display 220 is displayed based on the endoscope coordinate system C13 of the endoscope 3. In this embodiment, the image captured by the endoscope 3 is also displayed on the monitor 140 of the remote control apparatus 200. The image displayed on the monitor 140 is displayed based on the endoscope coordinate system C13 of the endoscope 3.

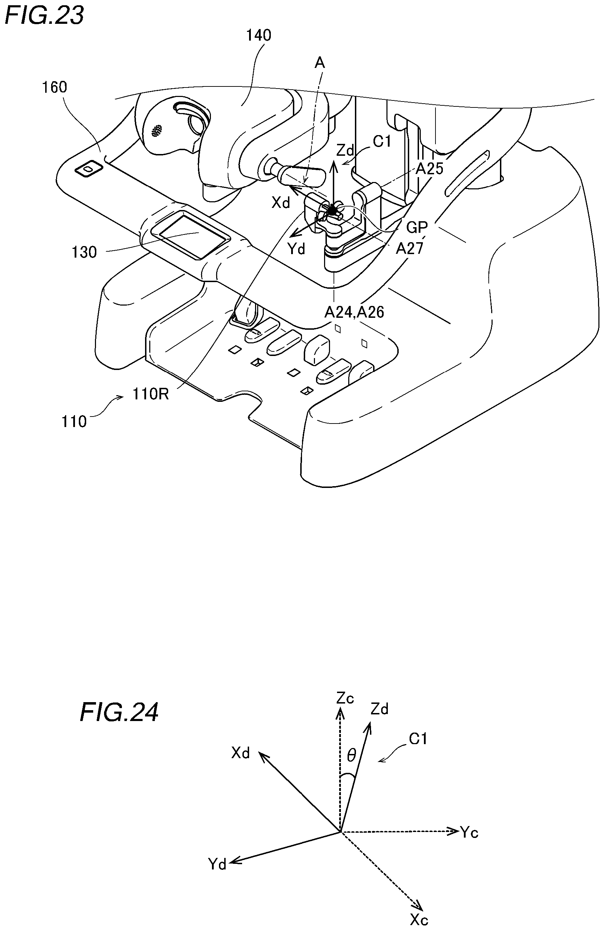

Next, the coordinate system C1 of the operation unit 110 is described. As shown in FIG. 23, a point at which the A24 axis, the A25 axis, the A26 axis, and the A27 axis of the operation unit 110 intersect with each other is called a gimbal point GP. The origin of the coordinate system C1 of the operation unit 110 is the gimbal point GP. The coordinate system C1 of the operation unit 110 is set individually for the right-handed operation unit 110R and the left-handed operation unit 110L. The three axes of the coordinate system C1 of the operation unit 110 are an Xd axis, a Yd axis, and a Zd axis.

In this embodiment, as shown in FIG. 24, the coordinate system C1 of the operation unit 110 is rotated by a predetermined angle θ with respect to the coordinate system of the surgical robot 100. Specifically, the coordinate system C1 of the operation unit 110 is the same as a coordinate system obtained by rotating the endoscope coordinate system C13 by 180 degrees around the Zc axis and rotating it by -90 degrees + the predetermined angle θ around the Xc axis. That is, as compared with the endoscope coordinate system C13 indicated by dotted arrows in FIG. 24, the Xd axis of the coordinate system C1 of the operation unit 110 and the Xc axis of the endoscope coordinate system C13 have opposite positive and negative directions. Furthermore, the Zd axis of the coordinate system C1 of the operation unit 110 is rotated by the predetermined angle θ around the Xc axis with respect to the Zc axis of the endoscope coordinate system C13. Moreover, the Xd axis of the coordinate system C1 of the operation unit 110 is along the right-left direction of the operator who operates the remote control apparatus 200. The Yd axis of the coordinate system C1 of the operation unit 110 is along the forward-rearward direction of the operator who operates the remote control apparatus 200. The endoscope coordinate system C13 is an example of a coordinate system of a surgical apparatus.

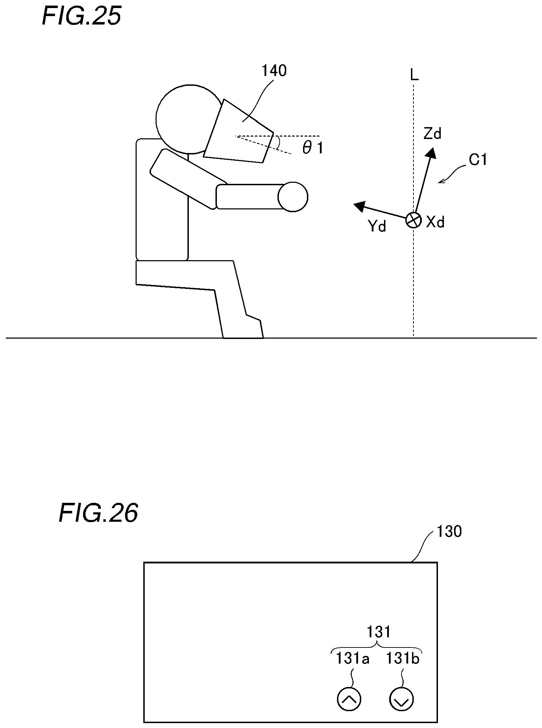

In this embodiment, as shown in FIG. 25, the predetermined angle θ of the coordinate system C1 of the operation unit 110 is an angle by which the coordinate system C1 of the operation unit 110 is rotated in a direction away from the operator with respect to a line L perpendicular to a surface on which the remote control apparatus 200 is placed. In other words, the coordinate system C1 of the operation unit 110 is rotated by the predetermined angle θ toward the far side rather than the near side as viewed from the operator.

In this embodiment, as shown in FIG. 23, the touch panel 130 of the remote control apparatus 200 receives a change in the predetermined angle θ. The predetermined angle θ can be changed in a range from 0 degrees to 50 degrees, for example. The touch panel 130 is arranged on the support bar 160. The touch panel 130 is arranged at a location corresponding to a location between the right-handed operation unit 110R and the left-handed operation unit 110L. In other words, the touch panel 130 is arranged in front of the operator. The touch panel 130 is an example of a receiver.

In this embodiment, as shown in FIG. 26, the touch panel 130 receives a change in the predetermined angle θ in fixed angular increments. For example, buttons 131 are provided on the touch panel 130 to change the predetermined angle θ. The buttons 131 include a button 131a to increase the predetermined angle θ and a button 131b to decrease the predetermined angle θ. Each time the button 131a is pressed once, the predetermined angle θ increases by one degree, for example. Each time the button 131b is pressed once, the predetermined angle θ decreases by one degree, for example. The variable range of the predetermined angle θ may be set. In such a case, a change in the predetermined angle θ is received within the variable range.

In this embodiment, the storage 311 shown in FIG. 15 stores the predetermined angle θ received for each operator. The first control device 310 retrieves the predetermined angle θ received for each operator and stored in the storage 311, and sets the coordinate system C1 of the operation unit 110 based on the retrieved predetermined angle θ. For example, an identifier such as an ID or a name for identifying the operator is registered by the operator operating the touch panel 130. After the operator operates the touch panel 130 to complete the change of the predetermined angle θ, the changed predetermined angle θ is stored in the storage 311 in association with the identifier. When the operator operates the touch panel 130 to select an identifier, the first control device 310 retrieves the predetermined angle θ associated with the identifier from the storage 311, and sets the coordinate system C1 of the operation unit 110 based on the retrieved predetermined angle θ.

In this embodiment, as shown in FIG. 23, the rotation angle of the monitor 140 of the remote control apparatus 200 is adjusted around an axis A along the right-left direction of the operator who operates the remote control apparatus 200. Specifically, the operator manually rotates the monitor 140 around the axis A with respect to the support arm 150. The touch panel 130 of the remote control apparatus 200 receives a change in the predetermined angle θ independently of the adjustment of the rotation angle of the monitor 140. That is, the rotation angle of the monitor 140 and the predetermined angle θ are not linked to each other and are adjusted separately. Therefore, it is possible to change the predetermined angle θ such that the rotation angle of the monitor 140 and the predetermined angle θ are equal to each other, and it is also possible to change the predetermined angle θ such that the rotation angle of the monitor 140 and the predetermined angle θ are different from each other.

### Control Method for Robotic Surgical System

A control method for the robotic surgical system 500 is now described.

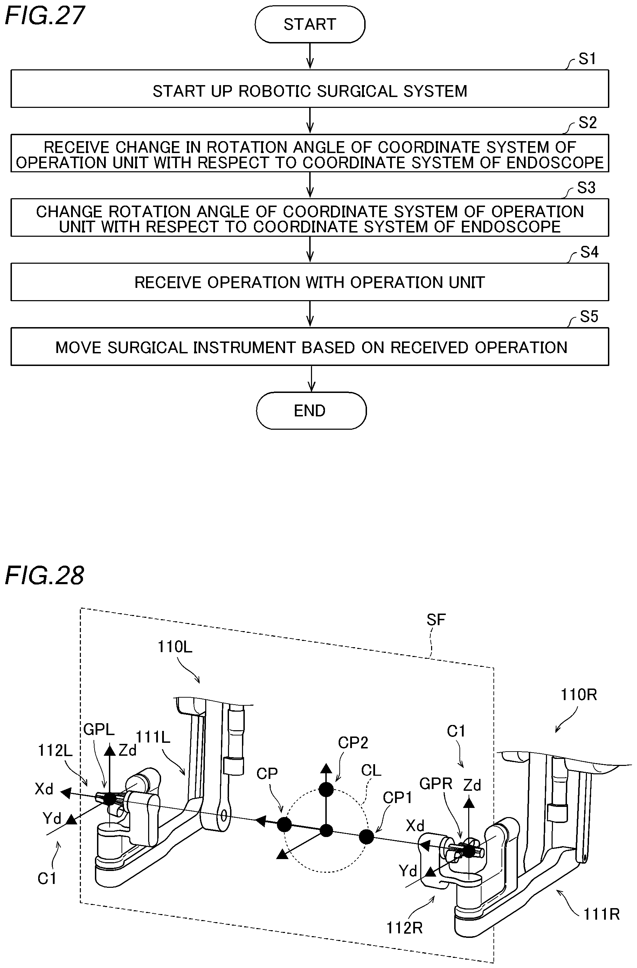

As shown in FIG. 27, in step S1, the robotic surgical system 500 is started up. The coordinate system C1 of the operation unit 110 is rotated by the predetermined angle θ with respect to the coordinate system of the surgical robot 100, and the predetermined angle θ is a predetermined value. The predetermined value is, for example, 15 degrees and is stored in the storage 311. The first control device 310 retrieves the predetermined value stored in the storage 311 and sets the coordinate system C1 of the operation unit 110.

In step S2, when the operator operates the touch panel 130, the first control device 310 receives a change in the predetermined angle θ by the operator.

In step S3, the first control device 310 changes the coordinate system C1 of the operation unit 110 based on the received change in the predetermined angle θ.

In step S4, the first control device 310 receives an operation with the operation unit 110 for the surgical instrument 1 attached to the robot arm 50 in a state in which the coordinate system C1 of the operation unit 110 has been changed. The operation of the operation unit 110 is performed by the operator.

An operation to move the endoscope 3 is described. While the camera pedal 124 of the foot pedals 120 shown in FIG. 14 is being pressed by the operator, the robot arm 50 to which the endoscope 3 has been attached can be operated with the operation unit 110. The endoscope 3 is moved by the operator operating both the right-handed operation unit 110R and the left-handed operation unit 110L. Specifically, as shown in FIG. 28, the first control device 310 defines a virtual plane SF based on the coordinate system C1 of the operation unit 110. A method for setting the virtual plane SF is as follows. The gimbal points GP of the operation units 110R and 110L are set as GPR and GPL, respectively. The midpoint of a line segment connecting the gimbal point GPR of the operation unit 110R to the gimbal point GPL of the operation unit 110L is set as CP. A point, the Y coordinate of which is the same as that of the midpoint CP and the X and Z coordinates of which are the same as those of a midpoint between the midpoint CP and the gimbal point GPR, is set as CP1. A straight line connecting the midpoint CP to the point CP1 is set as a diameter, and the intersection of a circle CL on a plane that passes through the same Y coordinate as the midpoint CP and the point CP1 with a line perpendicular to the straight line connecting the midpoint CP to the point CP1 is set as CP2. The virtual plane SF is a plane including CP, CP1, and CP2. When the predetermined angle θ of the coordinate system C1 of the operation unit 110 is changed, the rotation angle of the virtual plane SF around the Xd axis is also changed. The first control device 310 sets the movement amount of the midpoint CP in the previous control cycle and the current control cycle as the movement amount of the distal end of the endoscope 3. Specifically, the first control device 310 calculates a homogeneous transformation matrix that is a target for the position of the endoscope 3 in the current control cycle by adding the movement amount of the midpoint CP to a homogeneous transformation matrix that represents the position of the endoscope 3 in the previous control cycle. The first control device 310 performs an inverse kinematics calculation on the updated homogeneous transformation matrix. The first control device 310 calculates the axis values of the joint axes and the linear motion axes for the robot arm 50 and the surgical instrument 1 by the inverse kinematics calculation.

An operation to move the instrument 2 is described. The operator operates either the right-handed operation unit 110R or the left-handed operation unit 110L such that the robot arm 50 to which the instrument 2 has been attached can be operated. The first control device 310 calculates a homogeneous transformation matrix that is a target for the position of the instrument 2 in the current control cycle by adding the movement amount of the distal end of the instrument 2 to a homogeneous transformation matrix that represents the distal end position of the instrument 2 in the previous control cycle. The first control device 310 performs an inverse kinematics calculation on the updated homogeneous transformation matrix. The first control device 310 calculates the axis values of the joint axes and the linear motion axes for the robot arm 50 and the surgical instrument 1 by the inverse kinematics calculation. When the instrument 2 is moved, the coordinate system C1 of the operation unit 110 becomes the same as a coordinate system obtained by rotating the endoscope coordinate system C13 by 180 degrees around the Zc axis and rotating it by -90 degrees + the predetermined angle θ around the Xc axis as in the case when the endoscope 3 is moved.

In step S5, the first control device 310 moves the surgical instrument 1 by the robot arm 50 based on the received operation. Until the change of the predetermined angle θ is performed again, the operation in step S5 is continued based on the coordinate system C1 of the operation unit 110, the predetermined angle θ of which has been once changed.

In this embodiment, the second control device 350 monitors the command values of the first control device 310 for the robot arm 50 and the surgical instrument 1, and the actual axis values of the robot arm 50 and the surgical instrument 1. Specifically, during the operation period of step S5, the second control devices 350 monitors the command values of the first control device 310 and the actual axis values of the robot arm 50 and the surgical instrument 1 transmitted from the encoders E1, E2, and E3. The axis values are the rotation angles of the servomotors SM for the joint axes and the linear motion axes. When differences between the command values of the first control device 310 and the actual axis values of the robot arm 50 and the surgical instrument 1 exceed a predetermined threshold, the second control device 350 performs a control to issue a warning. For example, an alarm sound is generated from the speakers 22e of the medical cart 10 shown in FIG. 3.

### Advantages of This Embodiment

The touch panel 130 is configured to receive a change in the predetermined angle θ by the operator. Accordingly, the rotation angle of the coordinate system C1 of the operation unit 110 with respect to the coordinate system of the surgical robot 100 can be changed, and thus the coordinate system C1 of the operation unit 110 can be adjusted such that the operator can easily operate the operation unit 110. Consequently, the operability of the operation unit 110 can be improved.

The predetermined angle θ of the coordinate system C1 of the operation unit 110 is an angle by which the coordinate system C1 of the operation unit 110 is rotated in the direction away from the operator with respect to the line L perpendicular to the surface on which the remote control apparatus 200 is placed. Accordingly, the operator operates the operation unit 110 while looking down during surgery, and thus the angle of the coordinate system C1 of the operation unit 110 can be adjusted in a direction in which the operator looks down.

The touch panel 130 on the remote control apparatus 200 to receive settings for the robotic surgical system 500 is configured to receive a change in the predetermined angle θ. Accordingly, the change in the predetermined angle θ is received by the touch panel 130 provided on the remote control apparatus 200 in advance, and thus the complexity of the configuration of the robotic surgical system 500 can be reduced or prevented unlike a case in which a separate touch panel 130 is provided to receive a change in the predetermined angle θ.

The touch panel 130 is configured to receive a change in the predetermined angle θ in fixed angular increments. Accordingly, the amplitude of change in the predetermined angle θ is constant, and thus the possibility that the degree of change in the predetermined angle θ varies with each single operation by the operator can be reduced or prevented.

The surgical instrument 1 includes the endoscope 3. The robotic surgical system 500 includes the monitor 140 and the display 220 to display an image captured by the endoscope 3 in accordance with the endoscope coordinate system C13. Accordingly, the operator who operates the remote control apparatus 200 can appropriately adjust the coordinate system C1 of the operation unit 110 such that the operator can easily operate the operation unit 110 while visually checking the image captured by the endoscope 3 and displayed on the monitor 140. In addition, an assistant or the like other than the operator who operates the remote control apparatus 200 can visually check, on the display 220, the same image captured by the endoscope 3 that the operator visually checks on the monitor 140.

The robotic surgical system 500 includes the storage 311 to store the predetermined angle θ received for each operator. The first control device 310 is configured or programmed to retrieve the predetermined angle θ received for each operator and stored in the storage 311, and set the coordinate system C1 of the operation unit 110 based on the retrieved predetermined angle θ. Accordingly, the operator does not need to change the predetermined angle θ every time the robotic surgical system 500 is started up, and thus the need for the operator to change the predetermined angle θ can be eliminated.

The surgical instrument 1 includes the endoscope 3. The remote control apparatus 200 includes the monitor 140 to display an image captured by the endoscope 3 and allow the head of the operator to be inserted thereinto. The rotation angle of the monitor 140 is adjusted around the Xd axis along the right-left direction of the operator who operates the remote control apparatus 200, and the touch panel 130 is configured to receive a change in the predetermined angle θ independently of the adjustment of the rotation angle of the monitor 140. Accordingly, the predetermined angle θ can be changed to a desired angle such that each operator can more easily operate the operation unit 110 than when the predetermined angle θ of the coordinate system C1 of the operation unit 110 is automatically changed in conjunction with the change of the rotation angle of the monitor 140.

The second control device 350 is configured or programmed to monitor the command values of the first control device 310 for the robot arm 50 and the surgical instrument 1, and the actual axis values of the robot arm 50 and the surgical instrument 1. The second control device 350 is configured or programmed to perform a control to issue a warning when the differences between the command values of the first control device 310 and the actual axis values of the robot arm 50 and the surgical instrument 1 exceed the predetermined threshold. Accordingly, it is possible to reduce or prevent the possibility that the operator continues the operation in a state in which the command values of the first control device 310 are different from the actual axis values of the robot arm 50 and the surgical instrument 1 even when the predetermined angle θ of the coordinate system C1 of the operation unit 110 is changed.

### Modified Examples

The embodiment disclosed this time must be considered as illustrative in all points and not restrictive. The scope of the present disclosure is not shown by the above description of the embodiment but by the scope of claims for patent, and all modifications or modified examples within the meaning and scope equivalent to the scope of claims for patent are further included.

While the predetermined angle θ of the coordinate system C1 of the operation unit 110 is an angle by which the coordinate system C1 of the operation unit 110 is rotated in the direction away from the operator with respect to the line L perpendicular to the surface on which the remote control apparatus 200 is placed in the aforementioned embodiment, the present disclosure is not limited to this. In the present disclosure, the predetermined angle θ of the coordinate system C1 of the operation unit 110 may alternatively be an angle by which the coordinate system C1 of the operation unit 110 is rotated in a direction toward the operator with respect to the line L perpendicular to the surface on which the remote control apparatus 200 is placed.

While a change in the predetermined angle θ is received by the touch panel 130 of the remote control apparatus 200 in the aforementioned embodiment, the present disclosure is not limited to this. For example, a change in the predetermined angle θ may alternatively be received by an input other than the touch panel 130, such as a keyboard.

While the touch panel 130 receives a change in the predetermined angle θ in fixed angular increments in the aforementioned embodiment, the present disclosure is not limited to this. For example, the receiver that receives a change in the predetermined angle θ may alternatively be a rotary dial or slide switch, and a change in the predetermined angle θ may alternatively be received as a continuous value rather than in fixed angular increments.

While the coordinate system C1 of the operation unit 110 is rotated by the predetermined angle θ with respect to the endoscope coordinate system C13 in the aforementioned embodiment, the present disclosure is not limited to this. For example, the coordinate system C1 of the operation unit 110 may alternatively be rotated by the predetermined angle θ with respect to the coordinate system of the surgical robot 100 other than the endoscope coordinate system C13.

While the coordinate system C1 of the operation unit 110 is set based on the predetermined angle θ received for each operator and stored in the storage 311 in the aforementioned embodiment, the present disclosure is not limited to this. For example, the changed predetermined angle θ may not be stored in the storage 311.

While the second control device 350 monitors the command values of the first control device 310 and the actual axis values of the robot arm 50 and the surgical instrument 1, and performs a control to issue a warning when the differences between the command values of the first control device 310 and the axis values exceed the predetermined threshold in the aforementioned embodiment, the present disclosure is not limited to this. For example, the first control device 310 itself may alternatively monitor the command values of the first control device 310 and the actual axis values of the robot arm 50 and the surgical instrument 1, and perform a control to issue a warning when the differences between the command values of the first control device 310 and the axis values exceed the predetermined threshold.

While the remote control apparatus 200 includes two operation units 110 to operate two robot arms 50 in the aforementioned embodiment, the present disclosure is not limited to this. In the present disclosure, the remote control apparatus 200 may alternatively include only one operation unit 110 to operate one robot arm 50.

While as the controller according to the present disclosure, the first control device 310 arranged in the medical cart 10 is applied in the aforementioned embodiment, the present disclosure is not limited to this. In the present disclosure, a controller other than the first control device 310 may alternatively be applied as the controller according to the present disclosure.

While the touch panel 130 of the remote control apparatus 200 receives a change in the predetermined angle θ independently of adjustment of the rotation angle of the monitor 140 in the aforementioned embodiment, the present disclosure is not limited to this. In the present disclosure, the predetermined angle θ may alternatively be changed in conjunction with rotation of the monitor 140. The touch panel 130 may alternatively receive a further change in the predetermined angle θ that has been changed in conjunction with rotation of the monitor 140. Accordingly, the predetermined angle θ is automatically changed in conjunction with rotation of the monitor 140, and thus it is possible to save the operator time and effort. Furthermore, the touch panel 130 allows fine adjustment of the predetermined angle θ that has been changed in conjunction with rotation of the monitor 140.

While four robot arms 50 are provided in the aforementioned embodiment, the present disclosure is not limited to this. In the present disclosure, the number of robot arms 50 may alternatively be any number as long as there is at least one.

While each of the arm portion 51 and the positioner 30 includes a 7-axis articulated robot in the aforementioned embodiment, the present disclosure is not limited to this. For example, each of the arm portion 51 and the positioner 30 may alternatively include an articulated robot having an axis configuration other than the 7-axis articulated robot. The axis configuration other than the 7-axis articulated robot refers to six axes or eight axes, for example.

While the surgical robot 100 includes the medical cart 10, the positioner 30, the arm base 40, and the robot arms 50 in the aforementioned embodiment, the present disclosure is not limited to this. For example, the surgical robot 100 may not include the medical cart 10, the positioner 30, or the arm base 40, but may include only the robot arms 50.

The functionality of the elements disclosed herein may be implemented using circuitry or processing circuitry that includes general purpose processors, special purpose processors, integrated circuits, application specific integrated circuits (ASICs), conventional circuitry and/or combinations thereof that are configured or programmed to perform the disclosed functionality. Processors are considered processing circuitry or circuitry as they include transistors and other circuitry therein. In the present disclosure, the circuitry, units, or means are hardware that carries out the recited functionality or hardware that is programmed to perform the recited functionality. The hardware may be hardware disclosed herein or other known hardware that is programmed or configured to carry out the recited functionality. When the hardware is a processor that may be considered a type of circuitry, the circuitry, means, or units are a combination of hardware and software, and the software is used to configure the hardware and/or processor.

### Aspects

It will be appreciated by those skilled in the art that the exemplary embodiments described above are specific examples of the following aspects.

### (Item 1)

A robotic surgical system comprising:
a surgical apparatus including a robot arm to allow a surgical instrument to be attached thereto;
an operation apparatus including an operation unit to receive an operation for the surgical instrument, the operation unit having a coordinate system rotated by a predetermined angle with respect to a coordinate system of the surgical apparatus;
a controller configured or programmed to perform a control to move the surgical instrument by the robot arm based on the operation received by the operation unit; and
a receiver to receive a change in the predetermined angle by an operator.

### (Item 2)

The robotic surgical system according to item 1, wherein the predetermined angle is an angle by which the coordinate system of the operation unit is rotated in a direction away from the operator with respect to a line perpendicular to a surface on which the operation apparatus is placed.

### (Item 3)

The robotic surgical system according to item 1 or 2, wherein the receiver includes a touch panel on the operation apparatus to receive a setting for the robotic surgical system.

### (Item 4)

The robotic surgical system according to any one of items 1 to 3, wherein the receiver is configured to receive the change in the predetermined angle in fixed angular increments.

### (Item 5)

The robotic surgical system according to any one of items 1 to 4, wherein
the surgical instrument includes an endoscope; and
the robotic surgical system comprises a display to display an image captured by the endoscope in accordance with the coordinate system of the surgical apparatus.

### (Item 6)

The robotic surgical system according to any one of items 1 to 5, comprising:
a storage to store the predetermined angle received for each operator; wherein
the controller is configured or programmed to:
   retrieve the predetermined angle received for the each operator and stored in the storage; and
   set the coordinate system of the operation unit based on a retrieved predetermined angle.

### (Item 7)

The robotic surgical system according to any one of items 1 to 6, wherein
the surgical instrument includes an endoscope;
the operation apparatus includes a monitor to display an image captured by the endoscope and allow a head of the operator to be inserted thereinto;
the monitor has a rotation angle adjusted around an axis along a right-left direction of the operator who operates the operation apparatus; and
the receiver is configured to receive the change in the predetermined angle independently of adjustment of the rotation angle of the monitor.

### (Item 8)

The robotic surgical system according to any one of items 1 to 7, wherein
the surgical instrument includes an endoscope;
the operation apparatus includes a monitor to display an image captured by the endoscope and allow a head of the operator to be inserted thereinto;
the monitor has a rotation angle adjusted around an axis along a right-left direction of the operator who operates the operation apparatus;
the predetermined angle is changed in conjunction with rotation of the monitor; and
the receiver is configured to receive a further change in the predetermined angle that has been changed in conjunction with the rotation of the monitor.

### (Item 9)

The robotic surgical system according to any one of items 1 to 8, comprising:
a monitoring controller configured or programmed to monitor command values of the controller for the robot arm and the surgical instrument, and actual axis values of the robot arm and the surgical instrument; wherein
the monitoring controller is configured or programmed to perform a control to issue a warning when differences between the command values of the controller and the actual axis values exceed a predetermined threshold.

### (Item 10)

A control method for a robotic surgical system, the control method comprising:
receiving a change by an operator in a predetermined angle of a coordinate system of an operation unit rotated by the predetermined angle with respect to a coordinate system of a surgical apparatus;
changing the predetermined angle of the coordinate system of the operation unit based on a received change in the predetermined angle;
receiving an operation with the operation unit for a surgical instrument attached to a robot arm in a state in which the coordinate system of the operation unit has been changed; and
moving the surgical instrument by the robot arm based on a received operation.

### (Item 11)

A program for the control method for a surgical support system according to item 10.

### (Item 12)

A storage medium configured to store the program for the control method for a robotic surgical system according to item 11.

## Claims

1. A robotic surgical system (500) comprising:
a surgical apparatus (100) including a robot arm (50) to allow a surgical instrument (1) to be attached thereto;
an operation apparatus (200) including an operation unit (110) to receive an operation for the surgical instrument, the operation unit having a coordinate system (C1) rotated by a predetermined angle (θ) with respect to a coordinate system (C13) of the surgical apparatus;
a controller (310) configured or programmed to perform a control to move the surgical instrument by the robot arm based on the operation received by the operation unit; and
a receiver (130) to receive a change in the predetermined angle by an operator.

2. The robotic surgical system according to claim 1, wherein the predetermined angle is an angle by which the coordinate system of the operation unit is rotated in a direction away from the operator with respect to a line (L) perpendicular to a surface on which the operation apparatus is placed.

3. The robotic surgical system according to claim 1 or 2, wherein the receiver includes a touch panel (130) on the operation apparatus to receive a setting for the robotic surgical system.

4. The robotic surgical system according to any one of claims 1 to 3, wherein the receiver is configured to receive the change in the predetermined angle in fixed angular increments.

5. The robotic surgical system according to any one of claims 1 to 4, wherein
the surgical instrument includes an endoscope (3); and
the robotic surgical system comprises a display to display an image captured by the endoscope in accordance with the coordinate system of the surgical apparatus.

6. The robotic surgical system according to any one of claims 1 to 5, comprising:
a storage (311) to store the predetermined angle received for each operator; wherein
the controller is configured or programmed to:
retrieve the predetermined angle received for the each operator and stored in the storage; and
set the coordinate system of the operation unit based on a retrieved predetermined angle.

7. The robotic surgical system according to any one of claims 1 to 6, wherein
the surgical instrument includes an endoscope (3);
the operation apparatus includes a monitor (140) to display an image captured by the endoscope and allow a head of the operator to be inserted thereinto;
the monitor has a rotation angle adjusted around an axis along a right-left direction of the operator who operates the operation apparatus; and
the receiver is configured to receive the change in the predetermined angle independently of adjustment of the rotation angle of the monitor.

8. The robotic surgical system according to any one of claims 1 to 7, wherein
the surgical instrument includes an endoscope (3); the operation apparatus includes a monitor (140) to display an image captured by the endoscope and allow a head of the operator to be inserted thereinto;
the monitor has a rotation angle adjusted around an axis along a right-left direction of the operator who operates the operation apparatus;
the predetermined angle is changed in conjunction with rotation of the monitor; and
the receiver is configured to receive a further change in the predetermined angle that has been changed in conjunction with the rotation of the monitor.

9. The robotic surgical system according to any one of claims 1 to 8, comprising:
a monitoring controller (350) configured or programmed to monitor command values of the controller for the robot arm and the surgical instrument, and actual axis values of the robot arm and the surgical instrument; wherein
the monitoring controller is configured or programmed to perform a control to issue a warning when differences between the command values of the controller and the actual axis values exceed a predetermined threshold.

10. A control method for a robotic surgical system (500), the control method comprising:
receiving a change by an operator in a predetermined angle (θ) of a coordinate system (C1) of an operation unit (110) rotated by the predetermined angle with respect to a coordinate system (C13) of a surgical apparatus (100);
changing the predetermined angle of the coordinate system of the operation unit based on a received change in the predetermined angle;
receiving an operation with the operation unit for a surgical instrument (1) attached to a robot arm (50) in a state in which the coordinate system of the operation unit has been changed; and
moving the surgical instrument by the robot arm based on a received operation.

11. A program for the control method for a surgical support system (500) according to claim 10.

12. A storage medium configured to store the program for the control method for a robotic surgical system (500) according to claim 11.
